(19) Europäisches Patentamt
European Patent Office
Office européen des brevets

(11) Veröffentlichungsnummer: **0 015 498**
**A2**

# (12) EUROPÄISCHE PATENTANMELDUNG

(21) Anmeldenummer: **80100973.9**

(22) Anmeldetag: **27.02.80**

(51) Int. Cl.³: **C 07 D 249/08**, C 07 D 405/12, A 01 N 43/64 // (C07C69/653, 69/732)

(30) Priorität: **03.03.79 DE 2908324**

(43) Veröffentlichungstag der Anmeldung: **17.09.80 Patentblatt 80/19**

(84) Benannte Vertragsstaaten: **AT BE CH DE FR GB IT NL SE**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Ertel, Hartmut, Dr., Johann-Strauss-Strasse 24, D-6233 Kelkheim Taunus (DE)**
Erfinder: **Heubach, Günther, Dr., Luisenstrasse 15, D-6233 Kelkheim Taunus (DE)**
Erfinder: **Kocur, Jean, Dr., Pommernweg 14, D-6238 Hofheim am Taunus (DE)**
Erfinder: **Sachse, Burkhard, Dr., An der Ziegelei 30, D-6233 Kelkheim (Taunus) (DE)**

## (54) Triazolsubstituierte Hydratropasäurederivate, Verfahren zu ihrer Herstellung und ihre Verwendung als Pflanzenschutzmittel.

(57) Verbindungen der Formel I,

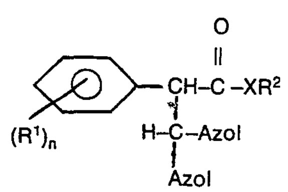

(I)

worin

$R^1$ Halogen, Phenyl, $C_{1-4}$-Alkyl, $C_{1-4}$-Alkoxy, $C_{1-3}$-Halogenalkoxy, $C_{1-4}$-Alkylthio oder die $CF_3$-Gruppe,

X Sauerstoff oder Schwefel,

$R^2$ $C_{1-8}$-Alkyl, das gegebenenfalls substituiert ist, $C_{2-8}$-Alkenyl, $C_{2-6}$-Alkinyl, $C_{3-8}$-Cycloalkyl, das gegebenenfalls substituiert ist, Phenyl, das gegebenenfalls substituiert ist, Benzyl, das gegebenenfalls substituiert ist, oder einen den Epoxiring enthaltenden Alkylrest mit bis zu 4 C-Atomen,

n 0, 1, 2 oder 3 und

Azol den 1,2,4-Triazol-1-yl-Rest 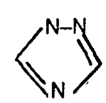

oder den 1,2,4-Triazol-4-yl-Rest 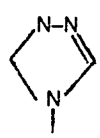

bedeuten, wobei die Verbindungen der Formel I auch in Form ihrer Salze mit anorganischen oder organischen Säuren oder als Metallkomplexverbindungen vorliegen können, ferner ein Verfahren zu ihrer Herstellung und ihre Verwendung als Schädlingsbekämpfungsmittel.

EP 0 015 498 A2

HOECHST AKTIENGESELLSCHAFT HOE 79/F 047 Dr.GM/Wa

# Triazolsubstituierte Hydratropasäurederivate, Verfahren zu ihrer Herstellung und ihre Verwendung als Pflanzenschutzmittel

Gegenstand der vorliegenden Erfindung sind neue, durch 1,2,4-Triazol substituierte Hydratropasäurederivate, ihre Herstellung und ihre Verwendung als Schädlingsbekämpfungsmittel im Pflanzenschutz.

Die erfindungsgemäßen Verbindungen besitzen die allgemeine Formel I,

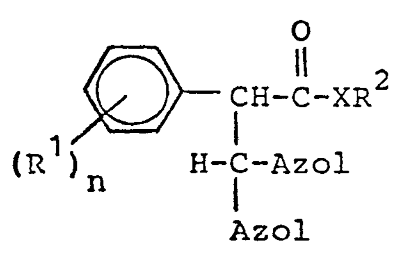

(I)

worin

$R^1$ Halogen, Phenyl, $C_{1-4}$-Alkyl, $C_{1-4}$-Alkoxy, $C_{1-3}$-Halogenalkc $C_{1-4}$-Alkylthio oder die $CF_3$-Gruppe,

X Sauerstoff oder Schwefel,

$R^2$ $C_{1-8}$-Alkyl, das gegebenenfalls durch Halogen, $C_{1-3}$-Alkoxyreste oder durch Alkoxycarbonylreste mit

bis zu 5 C-Atomen, oder durch Dialkylaminoreste mit bis zu 8 C-Atomen substituiert ist, $C_{2-6}$-Alkenyl, $C_{2-6}$-Alkinyl, $C_{3-8}$-Cycloalkyl, das gegebenenfalls durch $C_{1-4}$-Alkylreste substituiert ist, Phenyl, das gegebenenfalls durch Halogenatome, $C_{1-3}$-Alkylreste, $C_{1-3}$-Alkoxyreste oder die $CF_3$-Gruppe substituiert ist, Benzyl, das gegebenenfalls durch Halogen substituiert ist, oder einen den Epoxiring enthaltenden Alkylrest mit bis zu 4 C-Atomen,

n = O, 1, 2 oder 3 und

Azol den 1,2,4-Triazol-1-yl-Rest 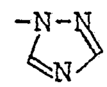
oder den 1,2,4-Triazol-4-yl-Rest 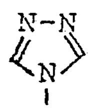

bedeuten.

Die Verbindungen der Formel I können auch in Form ihrer Salze mit anorganischen oder organischen Säuren, oder als Metallkomplexverbindungen, vorzugsweise Kupferkomplexverbindungen, vorliegen, wobei die letzteren in üblicher Weise, z.B. aus $CuCl_2$ und Verbindungen der Formel I erhalten werden können. Bevorzugt bedeuten in Formel I $R^1$ Halogen, insbesondere Chlor, X Sauerstoff, n 0 oder 1 und Azol den 1,2,4-Triazol-1-yl-Rest.

Die Verbindungen der Formel I können dadurch hergestellt werden, daß man Verbindungen der allgemeinen Formel II,

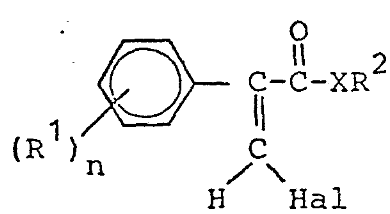
(II)

in der $R^1$, $R^2$, X und n die Bedeutungen wie in Formel I haben und Hal für Halogen, vorzugsweise Chlor oder Brom, insbesondere Chlor steht, bei höheren Temperaturen, vorzugsweise zwischen 60 und 120°C in Gegenwart eines Halogenwasserstoff bindenden Mittels, vorzugsweise

mindestens einer stöchiometrischen oder überschüssigen Menge dieses Mittels, beispielsweise einer basischen Verbindung, wie z.B. Kaliumcarbonat, Natriumcarbonat, Triethylamin oder N,N-Dimethylanilin entweder mit mindestens 2 Mol 1,2,4-Triazol pro Mol Verbindung der Formel II direkt zu den Verbindungen der Formel I umsetzt, oder mit mindestens einem Mol 1,2,4-Triazol pro Mol Verbindung der Formel II zunächst zu den ebenfalls neuen Monotriazolderivaten der Formel III umsetzt (vgl. Reaktionsschema 1). Diese Umsetzungen führt man vorteilhaft in inerten organischen Lösungsmitteln, wie z.B. Aceton, Acetonitril, Dimethylformamid oder Xylol durch.

Reaktionsschema 1

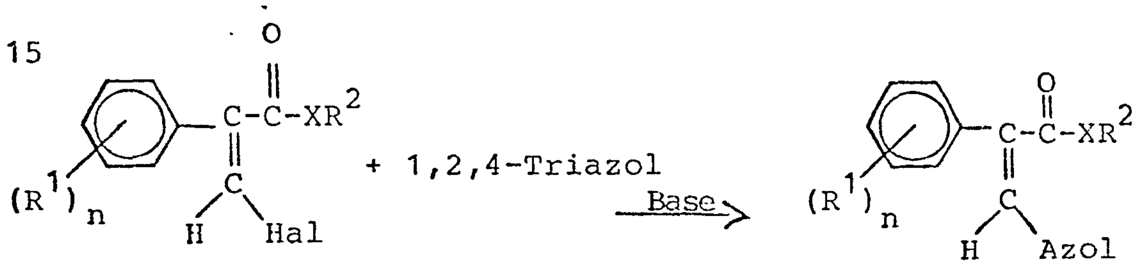


(II) (III)

Die Verbindungen der Formel III werden sodann entweder im isolierten Zustand, oder auch im Reaktionsgemisch mit mindestens einem weiteren Mol 1,2,4-Triazol pro Mol Verbindung der Formel III unter Zusatz einer katalytischen bis überschüssigen Menge einer anorganischen oder organischen Base, vorzugsweise Triethylamin, bei Temperaturen zwischen 20 und 120°C, vorzugsweise 60 bis 100°C, vorteilhaft in organischen Lösungsmitteln, nach Reaktionsschema 2 zu den erfindungsgemäßen Verbindungen der Formel I umgesetzt.

Reaktionsschema 2

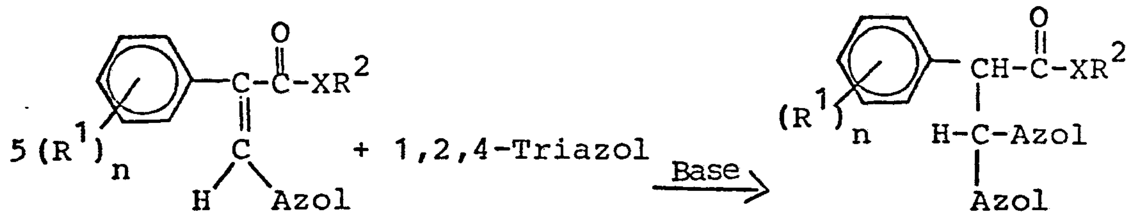


(III) (I)

Die zweistufige Umsetzung der Verbindungen der Formel II über die Zwischenstufe der Verbindungen der Formel III zu den Verbindungen der Formel I kann in manchen Fällen vorteilhaft sein, z.B. bei solchen Ausgangsverbindungen der Formel II, deren direkte, einstufige Umsetzung nur zu unbefriedigenden Ausbeuten an Verbindungen der Formel I führt.

Die zum Teil neuen Vorprodukte der Formeln IV und V können z.B. gemäß Reaktionsschema 3 bzw. 4 auf folgendem Weg erhalten werden:

Reaktionsschema 3

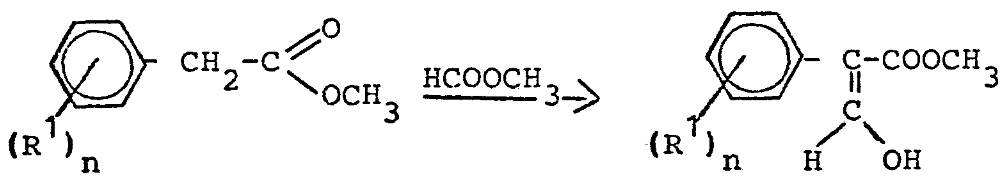


(IV)

Dieser Reaktionstyp ist in Liebigs Annalen der Chemie, 413, 206 (1917), beschrieben.

Nach Reaktionsschema 4 erhält man durch Chlorieren der Verbindungen der Formel IV die Produkte der Formel V. Die mit schlechten Ausbeuten verlaufende Chlorierung mit $PCl_5$ ist in Berichte dtsch. chem. Ges. 51, 1368 (1918) für n = O beschrieben. Die mit wesentlich besseren Ausbeuten

verlaufende Chlorierung mit $POCl_3$ in Dimethylformamid (DMF) ist noch nicht beschrieben. Anstelle von $POCl_3$ oder $PCl_5$ lassen sich z.B. auch Thionylchlorid, Phosphortrichlorid oder Phosgen verwenden.

Reaktionsschema 4

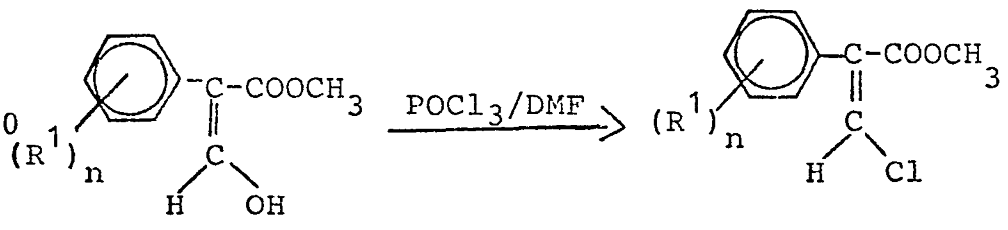


(IV) (V)

Höhere Ester lassen sich aus den Methylestern der Formel V nach bekannten Methoden durch Umesterung herstellen.

Ein weiterer Syntheseweg für höhere Ester (Va) ist in dem Reaktionsschema 5 skizziert.

Reaktionsschema 5

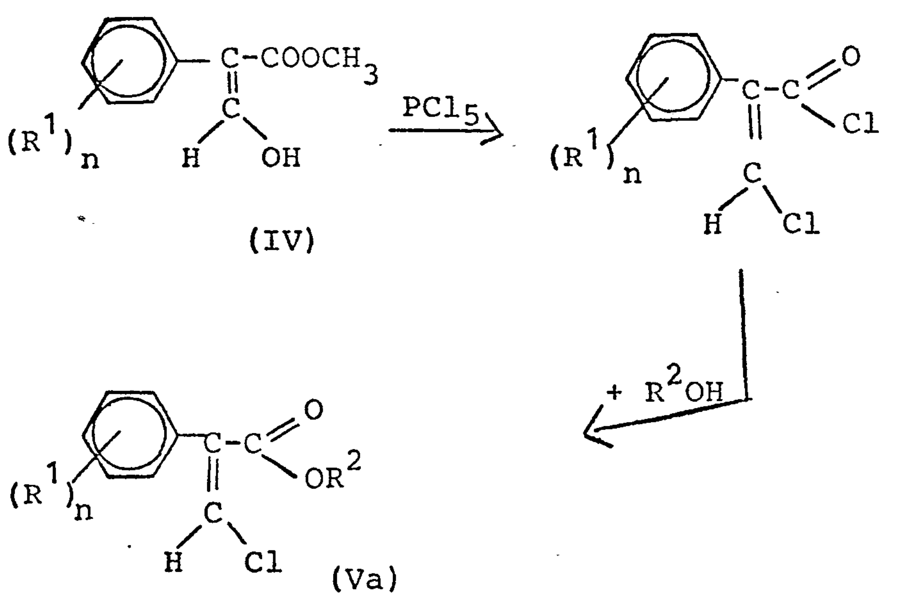


(IV) (VI) (Va)

Die Chlorierung IV — VI ist für n=O in Ber. dtsch. chem. Ges. 51, 1368 (1918) beschrieben.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt z.B. neben Piricularia oryzae und verschiedenen Rostarten vor allem Echte Mehltaupilze im Obst-, Gemüse-, Getreide- und Zierpflanzenanbau. Besonders hervorzuheben ist die ausgezeichnete Wirkung der Verbindungen gegen Mehltauarten, die gegen Benzimidazolderivate (z.B. Benomyl, Carbendazim) resistent sind.

Die Verbindungen der Formel I eignen sich auch für den Einsatz im technischen Bereich, beispielsweise in Holzschutzmitteln, auf dem Anstrichfarbensektor, als Konservierungsmittel, z.B. in Kühlschmiermitteln für die Metallbearbeitung.

Für die Anwendung im Pflanzenschutz können die fungiziden Verbindungen der Formel I in üblicher Weise als Stäube, Spritzpulver, Beizmittel, Dispersionen, Lösungen oder Emulsionskonzentrate formuliert werden. Der Gehalt an Wirkstoffen der Formel I liegt im allgemeinen zwischen 2 und 95 Gew.-%, vorzugsweise bei 10-90 Gew.-%. Daneben enthalten die genannten Wirkstoff-Formulierungen gegebenenfalls die jeweils üblichen Haft-, Netz-, Dispergier-, Emulgier-, Penetrations-, Lösungsmittel-, Füll- und Trägerstoffe.

Auch Mischungen oder Mischformulierungen mit anderen pestiziden Wirkstoffen, wie z.B. Insektiziden, Akariziden, Herbiziden oder Fungiziden sowie von Düngemitteln bzw. von Wachstumsregulatoren sind gegebenenfalls möglich.

Die Erfindung wird durch die nachfolgenden Beispiele

erläutert.

A. VORPRODUKTE für die Herstellungsbeispiele

1) 2-(4-Chlorphenyl)-3-chloracrylsäuremethylester

Zu einer aus 65,5 g (2,85 g-Atom) Natrium und 800 ml absolutem Toluol hergestellten Natriumsuspension läßt man ein Gemisch aus 504 g (2,73 Mol) p-Chlorphenylessigsäuremethylester, 164 g (2,73 Mol) Ameisensäuremethylester und 30 g Methanol so zulaufen, daß die Temperatur nicht über 40°C steigt. Nach beendetem Zulauf rührt man 4 Stunden bei 40°C und 12 Stunden bei Raumtemperatur. Das ausgefallene Natriumsalz des 2-(4-Chlorphenyl)-3-hydroxyacrylsäuremethylesters wird abgesaugt, mit 200 ml Toluol nachgewaschen, in 3 l Wasser eingetragen und mit verdünnter Schwefelsäure angesäuert (pH 2). Der 2-(4-Chlorphenyl)-3-hydroxyacrylsäuremethylester wird mit Methylenchlorid extrahiert, der Extrakt getrocknet und eingeengt. Man erhält 516 g eines semikristallinen Produktes, das ohne weitere Reinigung in 600 ml absolutem Dimethylformamid gelöst wird. Nach Zutropfen von 430 g (2,8 Mol) Phosphoroxychlorid bei 25°C läßt man 1 Stunde nachrühren und erwärmt dann 1 Stunde auf 110°C. Nach dem Abkühlen gießt man auf 4 l Eiswasser, extrahiert mit Methylenchlorid, trocknet den Extrakt, zieht das Lösungsmittel ab und destilliert den Rückstand. Man erhält 406 g 2-(4-Chlorphenyl)-3-chlor-acrylsäuremethylester, entsprechend einer Ausbeute von 64,4 % der Theorie.

$Kp_{1,8\ mbar}$ 121°C; $n_D^{22}$ 1,5615

2) 2-(4-Chlorphenyl)-3-chlor-acrylsäureisopropylester

115 g (0,5 Mol) 2-(4-Chlorphenyl)-3-chlor-acryl-

säuremethylester werden mit 108 g Isopropanol und 5,0 g Zirkon-(IV)-acetylacetonat 8 Stunden unter Rückfluß gehalten. Während dieser Zeit werden 300ml Alkohol abdestilliert und durch frisches Isopropanol ersetzt.
Danach wird unter Vakuum destilliert.

Man erhält 78 g 2-(4-Chlorphenyl)-3-chlor-acrylsäure-isopropylester vom $Kp_{0,1\ mbar}$ 92°C und $n_D^{22}$ 1,5395, entsprechend einer Ausbeute von 60,2 % der Theorie.

3) <u>2-(4-Chlorphenyl)-3-chlor-acrylsäureisobutylester</u>

115 g (0,5 Mol) 2-(4-Chlorphenyl)-3-chlor-acrylsäuremethylester werden mit 153 g Isobutanol und 4 g p-Toluolsulfonsäure 14 Stunden unter Rückfluß gekocht. Während dieser Zeit werden 5 mal 100 ml Alkohol abdestilliert und durch frisches Isobutanol ersetzt. Danach läßt man das Reaktionsgemisch abkühlen, zieht den überschüssigen Alkohol ab, nimmt den Rückstand in Methylenchlorid auf, wäscht die Lösung mit Wasser, trocknet sie, engt ein und destilliert. Man erhält 82 g 2-(4-Chlorphenyl)-3-chlor-acrylsäureisobutylester, entsprechend einer Ausbeute von 60 % der Theorie.
$Kp_{0,3\ mbar}$ 115-118°C; $n_D^{22}$ 1,5350.

4) <u>3-Chlor-2-phenyl-acrylsäure-ethylester</u>

Unter Rühren werden bei 40-45°C zu einer Mischung aus 140,8 g (0,733 Mol) 3-Hydroxy-2-phenyl-acrylsäure-ethylester und 53,5 g (0,735 Mol) Dimethylformamid 112,3 g (0,733 Mol) Phosphoroxychlorid zugetropft. Danach wird 90 Minuten bei 105°C nachgerührt. Nach dem Abkühlen wird das Gemisch in Eiswasser eingerührt und das abgeschiedene Öl mit Methylenchlorid aufgenommen. Nach dem Neutralwaschen

wird getrocknet und destilliert. Es werden 133,5 g Hauptfraktion aus 3-Chlor-2-phenyl-acrylsäure-ethylester erhalten, Kp 76-78°C/ 0,3 bis 0,4 mbar; Ausbeute: 86 % der Theorie.

5) 3-Hydroxy-2-phenyl-acrylsäure-ethylester

24 g Natrium (1,04 g-Atom) werden in 500 ml Toluol fein verteilt suspendiert. Bei 20-22°C wird ein Gemisch aus 164,1 g (1,0 Mol) Phenylessigsäureethylester und 74,1 g (1,0 Mol) Ameisensäureethylester unter Rühren und Eiswasserkühlung innerhalb von ca. 1 Stunde zugetropft und 5 Stunden nachgerührt. Nach 15 Stunden Stehen wird nochmals 1 Stunde auf 45°C erwärmt.

Das Reaktionsgemisch wird vorsichtig mit 1 Liter Eiswasser versetzt. Nach Phasentrennung wird die wäßrige Phase aufgearbeitet durch Ansäuern mit Essigsäure, Ausschütteln mit Methylenchlorid, Trocknen des Methylenchloridextrakts und Destillieren. Es werden 140,8 g 3-Hydroxy-2-phenyl-acrylsäure-ethylester gewonnen, $Kp_{0,4\ mbar}$: 78 - 79°C; Ausbeute: 73,3 % der Theorie.

B. HERSTELLUNGSBEISPIELE

Beispiel 1

2-(4-Chlorphenyl-3,3-bis-(1,2,4-triazol-1-yl)-propionsäure-isopropylester

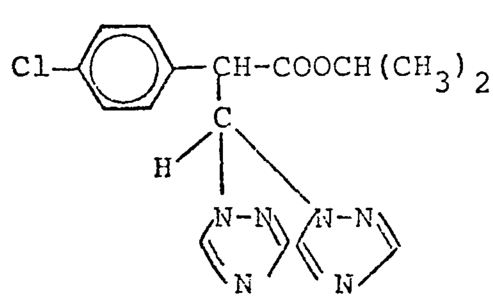

25 g (0,069 Mol) 2-(4-Chlorphenyl)-3-(1,2,4-triazol-1-yl)-acrylsäure-isopropylester werden mit 13,8 g (0,2 Mol) 1,2,4-Triazol in 100 ml Triethylamin 3 Stunden bei 80°C gerührt. Nach Abkühlen des Reaktionsgemisches wird das feste Produkt abgesaugt, getrocknet und 2 mal mit 80 ml Toluol digeriert. Man erhält 20 g eines farblosen Pulvers aus 2-(4-Chlorphenyl)-3,3-bis-(1,2,4-triazol-1-yl)-propionsäure-isopropylester vom Schmp. 172°C, entsprechend einer Ausbeute von 80,3 % der Theorie.

Analyse: $C_{16}H_{17}ClN_6O_2$ M = 360,8

ber.: C 53,26 %, H 4,75 %; N 23,29 %

gef.: C 53,1 %, H 4,5 %; N 23,0 %.

Beispiel 2

2-Phenyl-3,3-bis-(1,2,4-triazol-1-yl)-propionsäureethylester

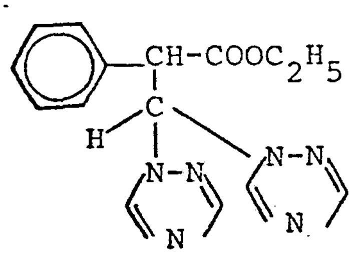

21,0 g (0,10 Mol) 3-Chlor-2-phenyl-acrylsäure-ethylester, 15,2 g (0,22 Mol) 1,2,4-Triazol und 17,9 g (0,13 Mol) Kaliumcarbonat werden in 100 ml Dimethylformamid 4 Stunden bei 82°C gerührt. Dann werden 30 ml Triethylamin und weitere 6,9 g (0,1 Mol) Triazol zugesetzt und nochmals 4 Stunden bei 80°C gerührt. Es entsteht eine orangegelbe Suspension. Nach dem Absaugen der Lösung vom Kaliumsalz wird der Hauptteil des Dimethylformamids unter Vakuum abdestilliert, der Rückstand mit Methylenchlorid verdünnt und mit Wasser gewaschen. Aus der getrockneten Methylenchloridlösung werden nach Abdestillieren des Methylenchlorids 35,8 g hellgelber, kristalliner Rückstand erhalten. Er wird mit 70 ml Toluol zerrieben, mit 70 ml Cyclohexan versetzt und abgesaugt. Es werden 20 g kristallines, farbloses Produkt aus 2-Phenyl-3,3-bis-(1,2,4-triazol-1-yl)-propionsäureethylester vom Fp. 158°C erhalten, entsprechend einer Ausbeute von 64,1 % der Theorie.

| | | |
|---|---|---|
| Analyse: | $C_{15}H_{16}N_6O_2$ | M = 312,3 |
| ber.: | C 57,68 %; H 5,16 %; N 26,91 % | |
| gef.: | C 57,70 %; H 4,90 %; N 26,60 % | |

Beispiel 3

2-(4-Chlorphenyl)-3,3-bis-(1,2,4-triazol-1-yl)-propionsäure-isobutylester

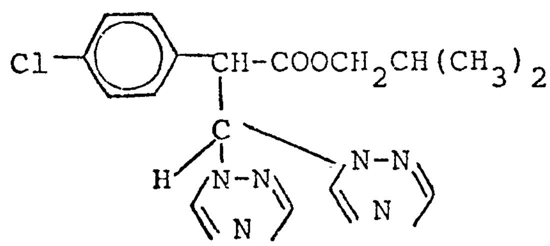

29,8 g (0,091 Mol) roher 2-(4-Chlorphenyl)-(1,2,4-triazol-1-yl)-acrylsäure-isobutylester werden mit 13,8 g (0,2 Mol) 1,2,4-Triazol in 100 ml Triethylamin 3 Stunden bei 80°C gerührt. Nach Abkühlen wird das Reaktionsgemisch am Rotavapor eingeengt, der Rückstand in 800 ml Methylenchlorid aufgenommen, die Lösung sorgfältig mit Wasser gewaschen, getrocknet und eingeengt. Der kristalline Rückstand wird mit 200 ml Toluol/Hexan = 1/1 digeriert und abgesaugt. Es verbleiben 26,9 g eines farblosen Pulvers aus 2-(4-Chlorphenyl)-3,3-bis-(1,2,4-triazol-1-yl)-propionsäure-isobutylester vom Fp. 163-165°C, entsprechend einer Ausbeute von 78,9 % der Theorie.

| | | | |
|---|---|---|---|
| Analyse: | $C_{17}H_{19}ClN_6O_2$ | M = 374,81 | |
| ber.: | C 54,47%; | H 5,07 %; | N 22,43 % |
| gef.: | C 54,10%; | H 5,10 %; | N 22,20 % |

Beispiele 4-39

In analoger Weise wie in Beispiel 1 beschrieben werden die Beispiele 4 bis 10 und 18 bis 22 ausgeführt, während die Beispiele 11 bis 17 und 29 bis 39 analog dem Beispiel 2 und die Beispiele 23 bis 28 analog dem Beispiel 3 ausgeführt werden. In der Tabelle 1 sind die Reste $R^1$ und $R^2$ sowie n in Formel I der nach den Beispielen 4 bis 39 aus den entsprechenden Verbindungen der Formel II hergestellten Verbindungen und deren Schmelzpunkte aufgeführt.

Tabelle 1

Formel I:

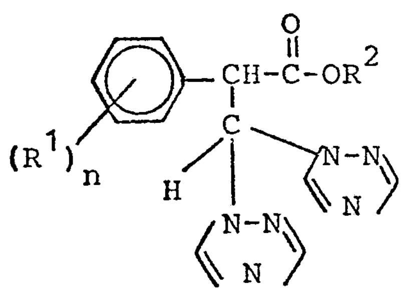

| Beispiel Nr. | $R^1$ | n | $R^2$ | Schmp. °C |
|---|---|---|---|---|
| 4 | | 0 | $-CH_3$ | 168-171 |
| 5 | | 0 | $-CH_2CH_2CH_3$ | 140-141 |
| 6 | | 0 | $-CH(CH_3)_2$ | 157 |
| 7 | | 0 | $-(CH_2)_3CH_3$ | 141 |
| 8 | | 0 | $-CH_2CH(CH_3)_2$ | 98-99 |
| 9 | | 0 | $-(CH_2)_4CH_3$ | 139-140 |
| 10 | | 0 | $-(CH_2)_2CH(CH_3)_2$ | 165 |
| 11 | | 0 | $-CH_2\underset{CH_3}{CH}-CH_2CH_3$ | 145-146 |

| Beispiel Nr. | $R^1$ | n | $R^2$ | Schmp. °C |
|---|---|---|---|---|
| 12 | | 0 | $-CH(CH_3)-CH_2CH(CH_3)_2$ | 191 |
| 13 | | 0 | $-(CH_2)_5CH_3$ | 108 |
| 14 | | 0 | $-CH_2-$ 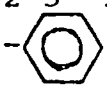 | 156 |
| 15 | | 0 | $-CH_2-CH=CH_2$ | 146-147 |
| 16 | | 0 | $-CH(CH_3)(CH_2)_2CH_3$ | 172-173 |
| 17 | 4-Cl | 1 | $-CH_3$ | 186-189 |
| 18 | 4-Cl | 1 | $-(CH_2)_4CH_3$ | 175 |
| 19 | 4-Cl | 1 | $-(CH_2)_5CH_3$ | 156 |
| 20 | 4-Cl | 1 | $-(CH_2)_3CH_3$ | 187 |
| 21 | 4-Cl | 1 | $-CH_2CH_2CH_3$ | 156 |
| 22 | 4-Cl | 1 | $-CH_2CH_2CH(CH_3)_2$ | 207-208 |
| 23 | 4-Cl | 1 | $-C_2H_5$ | 182 |
| 24 | 4-Cl | 1 | $-CH_2-$ 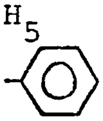 | 198-200 |
| 25 | 4-Cl | 1 | - 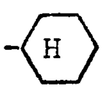 | 207 |
| 26 | 4-Cl | 1 | $-CH(CH_3)-CH_2CH(CH_3)_2$ | 221 |
| 27 | 4-Cl | 1 | $-CH(CH_3)-CH_2CH_2CH_3$ | 217 |
| 28 | | 0 | 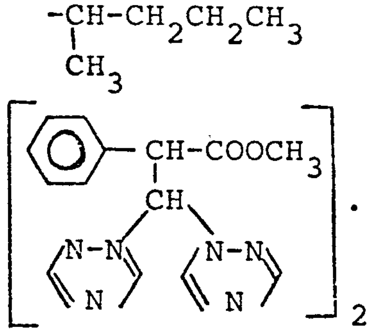 . $CuCl_2$ | 251 |
| 29 | 4-Cl | 1 | $-CH_2-CH=CH_2$ | 158 |
| 30 | 4- 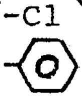 | 1 | $-CH_3$ | 219 |
| 31 | $3-OCF_2CF_2H$ | 1 | $-C_2H_5$ | 137 |
| 32 | $3-CF_3$ | 1 | $-CH_3$ | 153 |
| 33 | $3-CF_3$ | 1 | $-n-C_6H_{13}$ | 114 |
| 34 | | 0 | $-CH(CH_3)-C_2H_5$ | 158 |

| Beispiel Nr. | $R^1$ | n | $R^2$ | Schmp. °C |
|---|---|---|---|---|
| 35 | 2-Cl | 1 | $-CH_3$ | 144-145 |
| 36 | 2-Cl | 1 | $-C_2H_5$ | 115-119 |
| 37 | 3,4-$Cl_2$ | 2 | $-CH_3$ | 172-173 |
| 38 | 4-$CH_3O$ | 1 | $-CH_3$ | 184-186 |
| 39 | 4-$CH_3O$ | 1 | $-C_2H_5$ | 142-146 |

## C. FORMULIERUNGSBEISPIELE

### Beispiel A

Ein Stäubemittel wird erhalten, indem man
10 Gewichtsteile Wirkstoff und
90 Gewichtsteile Talkum als Inertstoff
mischt und in einer Schlagmühle zerkleinert.

0015498

Beispiel B

Ein in Wasser leicht dispergierbares benetzbares Pulver wird erhalten, indem man

25 Gewichtsteile Wirkstoff
64 Gewichtsteile kaolinhaltiger Quarz als Inertstoff
10 Gewichtsteile ligninsulfonsaures Kalium
und 1 Gewichtsteil oleoylmethyltaurinsaures Natrium als Netz- und Dispergiermittel

mischt und in einer Stiftmühle mahlt.

Beispiel C

Ein in Wasser leicht dispergierbares Dispersionskonzentrat wird erhalten, indem man

20 Gewichtsteile Wirkstoff mit
6 Gewichtsteilen Alkylphenolpolyglykoläther (Triton X 207)
3 Gewichtsteilen Isotridecanolpolyglykoläther (8 AeO)+)
und 71 Gewichtsteilen paraffinischem Mineralöl (Siedebereich z.B. ca. 255 bis über 377°C)

mischt und in einer Reibkugelmühle auf eine Feinheit von unter 5 Mikron vermahlt.

Beispiel D

Ein emulgierbares Konzentrat wird erhalten aus

15 Gewichtsteilen Wirkstoff
75 Gewichtsteilen Cyclohexanon als Lösungsmittel
und 10 Gewichtsteilen oxäthyliertes Nonylphenol (10 AeO)+) als Emulgator.

+) Anzahl AeO= Anzahl Äthylenoxydeinheiten im Polyglykoläthermolekül.

D. BIOLOGISCHE BEISPIELE

Die Buchstaben A, B und C stehen für die nachfolgend genannten handelsüblichen Vergleichsmittel:

A: Methyl-1-(butylcarbamoyl)-2-benzimidazolcarbamat (Benomyl)

B: N-Tridecyl-2,6-dimethyl-morpholin (Tridemorph)

C: 2-(sec.-Butyl)-4,6-dinitro-phenyl-(3-methyl-2-butenoat) (Binapacryl).

Beispiel I

Weizenpflanzen werden im 3-Blattstadium mit Konidien des Weizenmehltaus (Erysiphe graminis) stark inokuliert und in einem Gewächshaus bei 20°C und einer relativen Luftfeuchte von 90 - 95 % aufgestellt. 3 Tage nach Inokulation werden die Pflanzen mit den in Tabelle I aufgeführten Verbindungen in den Wirkstoffkonzentrationen von 500, 250, 125, 60, 30 und 15 mg/Liter Spritzbrühe tropfnaß gespritzt. Als Vergleich wird das Vergleichsmittel B in analoger Weise eingesetzt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle I zusammengefaßt.

Tabelle I

| Verbindung gemäß Beispiel Nr. | mit Weizenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 17 | 0 | 0 | 0 | 0 | 0 | 0 |
| 5 | 0 | 0-3 | 3 | 5-10 | 15 | |
| 2 | 0 | 0 | 0 | 0 | 0-3 | 5 |
| 7 | 0 | 0 | 0 | 0-3 | 5 | |
| 3 | 0 | 0 | 0 | 0 | 0-3 | 5 |
| Vergleichsmittel B | 3 phytotoxisch | 5 | 10 | 15 | 25 | |
| unbehand. infiz. Pflanzen | 100 | | | | | |

Beispiel II

Gerstenpflanzen werden im 3-Blattstadium mit Konidien des Gerstenmehltaus (Erysiphe graminis sp. hordei) stark inokuliert und in einem Gewächshaus bei 20°C und einer relativen Luftfeuchte von 90 - 95 % aufgestellt. 3 Tage nach Inokulation werden die Pflanzen mit den in Tabelle II aufgeführten Verbindungen in den Wirkstoffkonzentrationen von 500, 250, 125, 60, 30 und 15 mg/Liter Spritzbrühe tropfnaß gespritzt. Als Vergleich wird das Vergleichsmittel B in analoger Weise eingesetzt. Nach einer Inkubationszeit von 10 Tagen werden die Pflanzen auf Befall mit Gerstenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle II zusammengefaßt.

Tabelle II

| Verbindung gemäß Beispiel Nr. | mit Gerstenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 17 | 0 | 0 | 0 | 0 | 0 | 0 |
| 2 | 0 | 0 | 0 | 0 | 0-3 | 3 |
| 7 | 0 | 0 | 0 | 0-3 | 5 | |
| 3 | 0 | 0 | 0 | 0 | 0-3 | 3-5 |
| Vergleichsmittel B | 3 | 3-5 | 5 | 5-10 | 15 | |
| unbehand. infiz. Pflanzen | 100 | | | | | |

Beispiel III

Gurkenpflanzen (Sorte Delikateß) werden im 2-Blattstadium mit einer Konidiensuspension von Gurkenmehltau (Erysiphe cichoracearum) stark inokuliert. Nach einer Antrocknungszeit der Sporensuspension von 30 Minuten werden die Pflanzen in einem Gewächshaus bei 22°C und 90 % relativer Luftfeuchte aufgestellt. 3 Tage nach Infektion werden die Pflanzen mit den in Tabelle III genannten Verbindungen und Wirkstoffkonzentrationen tropfnaß gespritzt. Als Vergleich wird das Vergleichsmittel A in analoger Weise eingesetzt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall). Das Ergebnis ist in der Tabelle III zusammengefaßt.

Tabelle III

| Verbindung gemäß Beispiel Nr. | mit Gurkenmehltau befallene Blattfläche in % bei mg Wirkstoff/Liter Spritzbrühe | | | | | |
|---|---|---|---|---|---|---|
| | 500 | 250 | 125 | 60 | 30 | 15 |
| 17 | 0 | 0 | 0 | 0 | 0 | 0 |
| 5 | 0 | 0-3 | 3 | 5 | 15 | |
| 2 | 0 | 0 | 0-3 | 3 | 5 | 10 |
| 7 | 0 | 0-3 | 5 | 10 | 15 | |
| 3 | 0 | 0 | 0 | 0 | 3 | |
| Vergleichsmittel A | 3 | 5 | 10 | 15 | 25 | 35 |
| unbehand. infiz. Pflanzen | 100 | | | | | |

Beispiel IV

Gurkenpflanzen (Sorte Delikateß) werden im 2-Blattstadium mit einer Konidiensuspension eines Benomylresistenten Gurkenmehltaustammes (Erysiphe cichoracearum) stark inokuliert. Nach einer Antrocknungszeit der Sporensuspension von 30 Minuten werden die Pflanzen in einem Gewächshaus bei 22°C und 90 % relativer Luftfeuchte aufgestellt. 3 Tage nach Infektion werden die Pflanzen mit den in Tabelle IV genannten Verbindungen und Wirkstoffkonzentrationen tropfnaß gespritzt. Als Vergleich wird das Vergleichsmittel A in analoger Weise eingesetzt. Nach 10 Tagen erfolgt die Bonitur. Der Befallsgrad wird ausgedrückt in % befallender Blattfläche, bezogen auf unbehandelte, infizierte Kontrollpflanzen (= 100% Befall). Das Ergebnis ist in der Tabelle IV zusammengefaßt.

Tabelle IV

| Verbindung gemäß Beispiel Nr. | mit Gurkenmehltau (Benomyl-resistenter Stamm) befallene Blattfläche in % bei mg Wirkstoff/Liter Spritzbrühe | | | | | |
|---|---|---|---|---|---|---|
| | 500 | 250 | 125 | 60 | 30 | 15 |
| 17 | 0 | 0 | 0 | 0 | 0 | 0 |
| 5 | 0 | 0-3 | 3-5 | 5 | 15 | |
| 2 | 0 | 0 | 3 | 3-5 | 5 | 10 |
| 7 | 0 | 0-3 | 5 | 10 | 15 | |
| 3 | 0 | 0 | 0 | 0 | 3-5 | 5 |
| Vergleichsmittel A | 35 | 60 | 100 | 100 | 100 | 100 |
| unbehand. infiz. Pflanzen | 100 | | | | | |

PATENTANSPRÜCHE:

1. Verbindungen der allgemeinen Formel I,

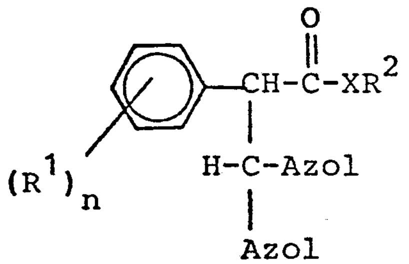

(I)

worin

$R^1$ Halogen, Phenyl, $C_{1-4}$-Alkyl, $C_{1-4}$-Alkoxy, $C_{1-3}$-Halogenalkoxy, $C_{1-4}$-Alkylthio oder die $CF_3$-Gruppe,

X Sauerstoff oder Schwefel,

$R^2$ $C_{1-8}$-Alkyl, das gegebenenfalls durch Halogen, $C_{1-3}$-Alkoxyreste oder durch Alkoxycarbonylreste mit bis zu 5 C-Atomen, oder durch Dialkylaminoreste mit bis zu 8 C-Atomen substituiert ist, $C_{2-8}$-Alkenyl, $C_{2-6}$-Alkinyl, $C_{3-8}$-Cycloalkyl, das gegebenenfalls durch $C_{1-4}$-Alkylreste substituiert ist, Phenyl, das gegebenenfalls durch Halogenatome, $C_{1-3}$-Alkylreste, $C_{1-3}$-Alkoxyreste oder die $CF_3$-Gruppe substituiert ist, Benzyl, das gegebenenfalls durch Halogen substituiert ist, oder einen den Epoxiring enthaltenden Alkylrest mit bis zu 4 C-Atomen,

n 0, 1, 2 oder 3 und

Azol den 1,2,4-Triazol-1-yl-Rest 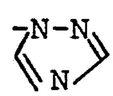 oder den 1,2,4-Triazol-4-yl-Rest 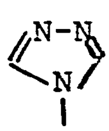 bedeuten.

Die Verbindungen der Formel I können auch in Form ihrer Salze mit anorganischen oder organischen Säuren oder als Metallkomplexverbindungen vorliegen.

0015498
HOE 79/F 047

2. Verfahren zur Herstellung von Verbindungen der Formel I gemäß Anspruch 1, dadurch gekennzeichnet, daß man Verbindungen der allgemeinen Formel II,

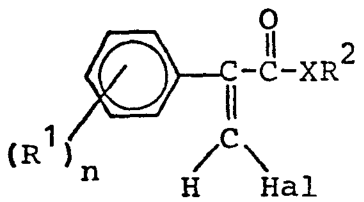

(II)

in der $R^1$, $R^2$, X und n die Bedeutungen wie in Formel I haben und Hal für Halogen steht, bei höheren Temperaturen, vorzugsweise zwischen 60 und 120°C, in Gegenwart eines Halogenwasserstoff bindenden Mittels entweder mit mindestens 2 Mol 1,2,4-Triazol pro Mol Verbindung der Formel II direkt zu den Verbindungen der Formel I, gegebenenfalls in inerten organischen Lösungsmitteln, umsetzt, oder mit mindestens einem Mol 1,2,4-Triazol pro Mol Verbindung der Formel II, gegebenenfalls in inerten organischen Lösungsmitteln, zunächst zu den Monotriazolderivaten der Formel III

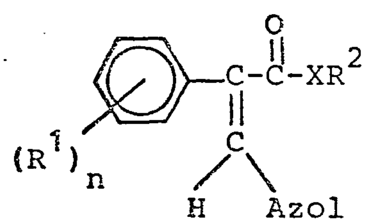

(III)

reagieren läßt und die Verbindungen der Formel III anschließend entweder im isolierten Zustand, gegebenenfalls in inerten organischen Lösungsmitteln, oder im Reaktionsgemisch mit mindestens einem weiteren Mol 1,2,4-Triazol pro Mol Verbindung der Formel III in Gegenwart einer katalytischen bis überschüssigen Menge einer anorganischen oder organischen Base bei Temperaturen zwischen 20 und 120°C zu den Verbindungen der Formel I umsetzt.

3. Schädlingsbekämpfungsmittel, gekennzeichnet durch einen Gehalt an einer Verbindung der Formel I gemäß Anspruch 1 als Wirkstoff.

4. Schädlingsbekämpfungsmittel, gekennzeichnet durch einen Gehalt von 2 bis 95 Gew.-% an einer Verbindung der Formel I gemäß Anspruch 1 als Wirkstoff sowie üblichen Formulierungshilfsmitteln.

5. Verwendung von Verbindungen der Formel I gemäß den Ansprüchen 1, 3 und 4 zur Schädlingsbekämpfung im Pflanzenschutz.

6. Verfahren zur Bekämpfung von Schadpilzen, dadurch gekennzeichnet, daß man die von ihnen befallenen Flächen und/oder Pflanzen und/oder Substrate mit einer fungizid wirksamen Menge von Wirkstoffen der Formel I gemäß Ansprüchen 1, 3 und 4 in Kontakt bringt.

Patentansprüche für Österreich

1. Verfahren zur Herstellung von Verbindungen der allgemeinen Formel I,

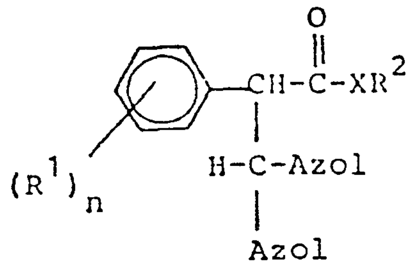

(I)

worin

$R^1$ Halogen, Phenyl, $C_{1-4}$-Alkyl, $C_{1-4}$-Alkoxy, $C_{1-3}$-Halogenalkoxy, $C_{1-4}$-Alkylthio oder die $CF_3$-Gruppe,

X Sauerstoff oder Schwefel,

$R^2$ $C_{1-8}$-Alkyl, das gegebenenfalls durch Halogen, $C_{1-3}$-Alkoxyreste oder durch Alkoxycarbonylreste mit bis zu 5 C-Atomen, oder durch Dialkylaminoreste mit bis zu 8 C-Atomen substituiert ist, $C_{2-8}$-Alkenyl, $C_{2-6}$-Alkinyl, $C_{3-8}$-Cycloalkyl, das gegebenenfalls durch $C_{1-4}$-Alkylreste substituiert ist, Phenyl, das gegebenenfalls durch Halogenatome, $C_{1-3}$-Alkylreste, $C_{1-3}$-Alkoxyreste oder die $CF_3$-Gruppe substituiert ist, Benzyl, das gegebenenfalls durch Halogen substituiert ist, oder einen den Epoxiring enthaltenden Alkylrest mit bis zu 4 C-Atomen,

n 0, 1, 2 oder 3 und

Azol den 1,2,4-Triazol-1-yl-Rest 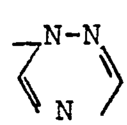 oder den 1,2,4-Triazol-4-yl-Rest 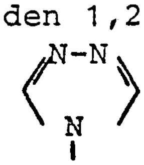

bedeuten, wobei die Verbindungen der Formel I auch in die Form ihrer Salze mit anorganischen oder organischen Säuren oder in Metallkomplexverbindungen überführt werden

können, dadurch gekennzeichnet, daß man Verbindungen der allgemeinen Formel II,

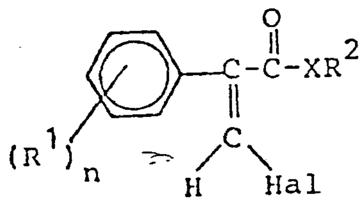

(II)

in der $R^1$, $R^2$, X und n die Bedeutungen wie in Formel I haben und Hal für Halogen steht, bei höheren Temperaturen in Gegenwart eines Halogenwasserstoff bindenden Mittels entweder mit mindestens 2 Mol 1,2,4-Triazol pro Mol Verbindung der Formel II direkt zu den Verbindungen der Formel I, gegebenenfalls in inerten organischen Lösungsmitteln, umsetzt, oder mit mindestens einem Mol 1,2,4-Triazol pro Mol Verbindung der Formel II, gegebenenfalls in inerten organischen Lösungsmitteln, zunächst zu den Monotriazolderivaten der Formel II

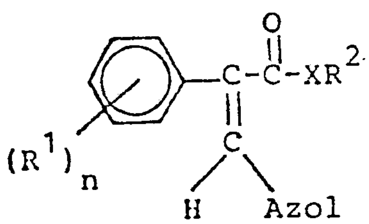

(III)

reagieren läßt und die Verbindungen der Formel III anschließend entweder im isolierten Zustand, gegebenenfalls in inerten organischen Lösungsmitteln, oder im Reaktionsgemisch mit mindestens einem weiteren Mol 1,2,4-Triazol pro Mol Verbindung der Formel III in Gegenwart einer katalytischen bis überschüssigen Menge einer anorganischen oder organischen Base bei Temperaturen zwischen 20 und 120°C zu den Verbindungen der Formel I umsetzt und diese gegebenenfalls in Salze mit anorganischen oder organischen Säuren oder in Metallkomplexverbindungen überführt.

2. Schädlingsbekämpfungsmittel, gekennzeichnet, durch einen Gehalt an einer Verbindung der Formel I gemäß Anspruch 1 als Wirkstoff.

3. Schädlingsbekämpfungsmittel, gekennzeichnet durch einen Gehalt von 2 bis 95 Gew.-% an einer Verbindung der Formel I gemäß Anspruch 1 als Wirkstoff sowie üblichen Formulierungshilfsmitteln.

4. Verwendung von Verbindungen der Formel I gemäß den Ansprüchen 1, 2 und 3 zur Schädlingsbekämpfung im Pflanzenschutz.

5. Verfahren zur Bekämpfung von Schadpilzen, dadurch gekennzeichnet, daß man die von ihnen befallenen Flächen und/oder Pflanzen und/oder Substrate mit einer fungizid wirksamen Menge von Wirkstoffen der Formel I gemäß Ansprüchen 1, 2 und 3 in Kontakt bringt.